# EUROPEAN PATENT SPECIFICATION

(11) **EP 1 914 300 B1**
(45) Date of publication and mention of the grant of the patent: **21.08.2013**
(21) Application number: 06781543.1
(22) Date of filing: 25.07.2006
(51) Int. Cl.: A61L 27/38

(54) **TOOTH REGENERATION METHOD**
ZAHNREGENERIERUNGSVERFAHREN
PROCEDE DE REGENERATION DENTAIRE

(30) Priority: 29.07.2005 JP 2005221668
(43) Date of publication of application: 23.04.2008
(73) Proprietor: Matsumoto Dental University, Shiojiri-shi, Nagano 399-0781 (JP)
(72) Inventor: YAMAKI, Mariko, Matsumoto Dental University, Shiojiri-shi, Nagano 399-0781 (JP); OZAWA, Hidehiro, Matsumoto Dental University, Shiojiri-shi, Nagano 399-0781 (JP); EBINA, Satoshi, The University of Tokyo, Tokyo 153-8902 (JP)
(74) Representative: Lord, Hilton David
(86) International application number: PCT/JP2006/314631
(87) International publication number: WO 2007/013430

(56) References cited:
- WO-A-03/004605
- WO-A1-01/60981
- FR-A- 2 784 284
- THESLEFF IRMA: "Epithelial-mesenchymal signalling regulating tooth morphogenesis." JOURNAL OF CELL SCIENCE, vol. 116, no. 9, 1 May 2003 (2003-05-01), pages 1647-1648, XP002548751 ISSN: 0021-9533
- KOYAMA E ET AL: "Chick limbs with mouse teeth: An effective in vivo culture system for tooth germ development and analysis" DEVELOPMENTAL DYNAMICS 20030101 US, vol. 226, no. 1, 1 January 2003 (2003-01-01), pages 149-154, XP002548752 ISSN: 1058-8388
- SHI S ET AL: "The efficacy of mesenchymal stem cells to regenerate and repair dental structures." ORTHODONTICS & CRANIOFACIAL RESEARCH AUG 2005, [Online] vol. 8, no. 3, August 2005 (2005-08), pages 191-199, XP002548866 ISSN: 1601-6335 [retrieved on 2005-07-15]
- RISBUD MV, SHAPIRO IM: "Stems cells in craniofacial and dental tissue engeneering" ORTHOD CRANIOFACIAL RES, [Online] vol. 8, pages 54-59, XP002548867 Retrieved from the Internet: URL:http://www3.interscience.wiley.com/cgi -bin/fulltext/120716509/PDFSTART> [retrieved on 2005-05-11]
- JERNVALL J. AND THESLEFF I.: 'Reiterative signaling and patterning during mammalian tooth morphogenesis' MECHANISMS OF DEVELOPMENT vol. 92, 2000, pages 19 - 29, XP003007644
- OHAZAMA A. ET AL.: 'Stem-cell-based tissue engineering of murine teeth' J. DENT. RES. vol. 83, no. 7, 2004, pages 518 - 522, XP003007645
- MODINO S.A.C. AND SHARPE P.T.: 'Tissue engineering of teeth using adult stem cells' ARCH. ORAL. BIOL. vol. 50, February 2005, pages 255 - 258, XP004751487
- YOUNG C.S. ET AL.: 'Tissue engineering of complex tooth structures on biodegradative polymer scaffolds' J. DENT. RES. vol. 81, no. 10, 2002, pages 695 - 700, XP003007646
- YAMAKI M. ET AL.: 'Mouse Kanyokei Saibo Chimera haiyotai o Mochiita in vitro to in vivo deno Ha no Saisei' 28TH ANNUAL MEETING OF THE MOLECULAR BIOLOGY SOCIETY OF JAPAN KOEN YOSHISHU 25 November 2005, page 676 (ABSTRACT 3P-0668), XP003007647

## Description

### TECHNICAL FIELD

The present invention relates generally to a method of regenerating a tooth of a mammal in vitro, and particularly to a method of forming a tooth using a chimera embryoid body of an undifferentiated cell and a dental mesenchymal cell derived from a non-human mammal of the same species as that of the target mammal. More particularly, the present invention relates to regenerative medicine, dental regenerative medicine, and pharmaceutical development techniques using the above method.

### BACKGROUND ART

For many years and continuing, attempts have been made to regenerate a mammal tooth in vitro, or regenerate a human tooth using knowledge and insight obtained from analyzing the regeneration mechanism of a mammal tooth, for example. It is noted that a tooth is formed by an epithelial cell (dental epithelium) derived from the ectoderm and a mesenchymal cell (dental mesenchyme) derived from the head neural crest. The dental epithelium corresponds to an ingrown portion of buccal cavity epithelium formed during the initial embryonic stage, and the dental mesenchyme corresponds to mesenchymal cells including undifferentiated cells derived from the neural crest that are accumulated below the dental epithelium. The interaction between the dental epithelium and the mesenchymal cell plays an important role in all stages of a tooth formation process (e.g., see below Non-Patent Document 1). Induction factors such as members of the fibroblast growth factor (FGF) family (e.g., see below Non-Patent Document 2), bone-forming factor 4 (BMP4), and Sonic Hedgehog (SHH) induce expression of homeobox genes such as Msx1, Msx2, Dlx1, Dlx2, Lef1, and Barx1 in the dental mesenchymal cells, and as a result, in the case of a mouse, it is believed that the dental mesenchymal cells acquire functions for determining the shape of the dental crown and the position of the tooth and functions for controlling differentiation of epithelium cells in eleven to fourteen embryonal days (ED11-14) (e.g., see below Non-Patent Document 3). SHH is a secretory protein and is known to be a factor related to body axis formation. In the tooth formation process, dental epithelium cells are secreted, and the SHH is believed to be an important factor for conveying various signals to the dental epithelium cells via the BMP (e.g., see below Non-Patent Document 4). Many tooth regeneration experiments have been conducted including those involving directly transplanting a tooth, a tooth germ, or dental pulp of a mouse or a rat into the living body; cultivating tissues of the tooth (organ culture) and transplanting the cultivated tissues into the living body; or separating the tooth into single cell units and cultivating the separated cells for use in the tooth regeneration process. However, there is no concrete evidence of successful formation of a tooth in any of these experiments. It is noted that experiments conducted in the past may be divided into the following three categories:
(1): Method using tissues involved in tooth formation within the living body (e.g., dental papilla, dental pulp, periodontal membrane)
   (1)-1. Directly transplant tissues of tooth germ, dental pulp, periodontal membrane, and the like (e.g., see below Non-Patent Document 5)
   (1)-2. Attempt tooth formation in vitro by separating tooth germ into epithelium cells and mesenchymal cells, cultivating each of the cell groups, inducing differentiation of each cell group into ameloblast and odontoblast
   (1)-3. Attempt tooth formation by remixing dental epithelium cells and mesenchymal cells that have been separated and cultivated and transplanting the remixed cells in a living body such as the skin of a mouse (e.g., see below Non-Patent Document 6) (2): Method using undifferentiated cells having multipotency such as embryonic stem cells or mesenchymal stem cells

Cultivate undifferentiated cells in a Petri dish with a suitable induction factor and induce tooth formation using a three dimensional matrix (3): Method using both of the methods (1) and (2)

The above method (3) is used in more recent attempts at tooth formation compared to the methods (1) and (2). It is noted that there have been reports of actual tooth formation by removing the dental epithelium from a 10-embryonal day (ED10) mouse fetus through surgery, layering the removed dental epithelium on a stem cell such as an embryonic stem cell or a bone marrow stem cell of a mouse, cultivating the same in the presence of FGF8 or BMP4, transplanting the cultivated cell into the upper jaw of a mouse (e.g., see below Non-Patent Documents 7 and 8). However, as is described in detail below, such a case cannot be considered a completely successful attempt at tooth formation since there is no evidence of enamel matrix formation in this case.

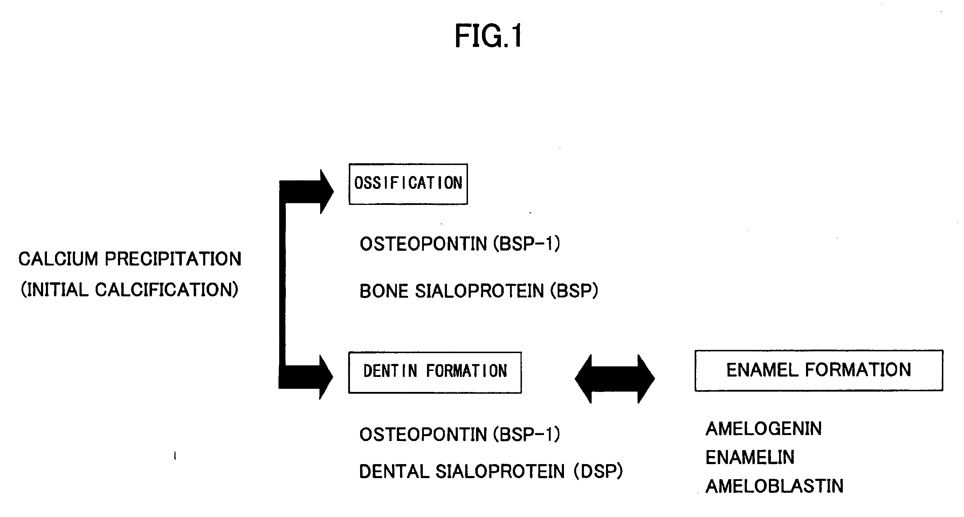

It is noted that histologically proving tooth formation may involve confirming the following: (a) calcium precipitation (calcification; namely, dentin formation); (b) enamel matrix formation; and (c) cement formation. Enamel is an important element for retaining hardness and cavity resistance of a tooth and is not regenerated after tooth formation. To confirm dentin formation, tissue staining such as the von Kossa staining method or the Alizarin red S staining method are used to detect gene and protein expressions of bone sialoprotein (BSP), osteopontin (OPN; BSP-1), dental sialoprotein (DSP), and the like. To confirm enamel matrix formation, protein and gene expressions of amelogenin, enamelin, ameloblastin and the like as components of the enamel matrix have to be detected (see FIG. 1). In the field of tooth regeneration, there have been a relatively large number of cases reporting calcification (dentin formation) and ossification. However, there have been no cases confirming enamel matrix formation; that is, expressions of amelogenin, enamelin, ameloblastin, and the like have not been detected.

Enamel is an important element for retaining hardness and cavity resistance of a tooth and cannot be regenerated after tooth formation. As is described above, enamel formation may be confirmed by detecting gene or protein expressions of amelogenin, enamelin, ameloblastin, and other components included in enamel. Since enamel formation may constitute evidence of tooth formation, confirmation of the existence of amelogenin, enamelin, and ameloblastin may be the key to successful tooth regeneration.

As is described above, one method of regenerating a tooth may involve the use of undifferentiated cells such as embryonic stem cells or somatic stem cells. Undifferentiated cells that have multipotency may develop into tumors upon being transplanted into the living body in their undifferentiated states. However, under suitable conditions such cells may be cultivated and increased to be induced to develop into various organs and tissues depending on the added induction factor. Therefore, research and development are vigorously being conducted on the use of undifferentiated cells having multipotency as the source cell for tooth regeneration. In recent years, much attention is being drawn to tooth regeneration engineering actively using undifferentiated cells having multipotency such as embryonic stem cells, somatic stem cells and bone marrow stem cells (e.g., see below Non-Patent Documents 7 and 8).

However, in previous cases, formation of enamel matrices such as amelogenin, enamelin, and ameloblastin that are essential for tooth formation have not been confirmed neither in vivo nor in vitro.
(Non-Patent Document 1): I. Thesleff. Epithelial-mesenchymal signaling regulating tooth morphogenesis. J. Cell Sci. (2003), 116, 1647-1648.
(Non-Patent Document 2): M. Mandler, A. Neubuser. FGF signaling is necessary for the specification of the odontogenic mesenchyme. Dev. Biol. (2001) 240, 548-559.
(Non-Patent Document 3): A.S. Tucker, K.L. Matthews, P.T. Sharpe. Transformation of tooth type induced by inhibition of BMP signaling. Science (1998) 282, 1136-1138.
(Non-Patent Document 4): P. Maye, S. Becker, H. Siemen, J. Thorne, N. Byrd, J. Carpentino, L. Grabel. Hedgehog signaling is required for the differentiation of ES cells into neuroectoderm. Dev. Biol. (2004) 265, 276-290.
(Non-Patent Document 5): E. Koyama, C. Wu, T. Shimo, M. Pacifici. Chick limbs with mouse teeth: an effective in vivo culture system for tooth germ development and analysis. Dev. Dyn. (2003) 226, 149-154.
(Non-Patent Document 6): M.J. Tabata, T, Matsumura, T. Fujii, M. Abe, K. Kurisu. Fibronectin accelerates the growth and differentiation of ameloblast lineage cells in vitro. J. Histol. Cyto. (2003) 51, 1673-1679.
(Non-Patent Document 7): A. Ohazama, S.A. Modino, I. Miletich, P.T. Sharpe. Stem-cell-based tissue engineering of murine teeth. J. Dent. Res. (2004) 83, 518-522.
(Non-Patent Document 8): S.A.C. Mondino, P.T. Sharpe. Tissue engineering of teeth using adult stem cells. Archiv. Oral Biol. (2005) 50, 255-258.

### DISCLOSURE OF THE INVENTION

An aspect of the present invention is directed to providing a novel in vitro tooth formation method that involves producing a chimera embryoid body using an undifferentiated cell and a dental mesenchymal cell derived from a non-human mammal of the same species as that of the target mammal and then cultivating the chimera embryoid body on a three-dimensional matrix.

In the living body of a mammal, a tooth is formed through interaction between an epithelial cell (dental epithelium) derived from the ectoderm and a mesenchymal cell (dental mesenchyme) derived from the head neural crest at all stages of the tooth formation process. Therefore, it has been believed that an epithelial cell line and a mesenchymal cell line derived from the tooth germ are necessary for tooth formation. However, it is difficult for dental epithelial cells to develop into a stable cell line independently on their own. Also, complete tooth regeneration has not yet been achieved using the tooth germ itself, the dental epithelium, dental mesenchymal cells, or a combined cell group of dental epithelium and dental mesenchymal cells. Accordingly, inventors of the present invention have directed their attention to testing results revealing the fact that dental mesenchymal cells can be developed into a cell line that retains undifferentiated cells and epithelial cell support inducing characteristics. Based on knowledge of the fact that differentiation of dental epithelial cells may be induced by using embryonic stem cells of a mouse in place of dental epithelial cells and causing interaction with dental mesenchymal cells, the inventors have developed a unique tooth formation method that involves cultivating embryonic stem cells in the presence of dental mesenchymal cells, separating the mixed cells, disseminating the separated cells on a low binding plastic plate to produce an chimera embryoid body, and further cultivating the chimera embryoid body on a three dimensional matrix.

A method of producing a chimera embryoid body involves co-cultivating an undifferentiated cell and a dental mesenchymal cell of a non-human mammal of a same species in the presence of an induction factor, wherein the induction factor is at least one of activin, bone morphogenic protein 4, insulin growth factor 1, fibroblast growth factor 2, or transforming growth factor β1.

An undifferentiated cell such as an embryonic cell and a dental mesenchymal cell of a non-human mammal of the same species such as a mouse may be co-cultivated in the presence of a suitable induction factor to produce a chimera embryoid body.

In the above method of producing a chimera embryoid body , the non-human mammal may be one species selected from all non-human mammal species.

A chimera embryoid body may be produced using a stem cell of any species of a non-human mammal including that of a mouse.

In the above method of producing a chimera embryoid body, the undifferentiated cell may be one type of stem cell selected from all types of stem cells.

A chimera embryoid body may be produced using any type of stem cell including an embryonic stem cell.

A chimera embryoid body is provided that is produced using the method according to any one of the above first through third embodiments.

A chimera embryoid body may be produced by co-cultivating, in the presence of a suitable induction factor, a dental mesenchymal cell and a stem cell of the same non-human mammal species that is selected from all non-human mammal species including a mouse, the stem cell being selected from all types of stem cells including the embryonic stem cell.

A cultivation method is provided that uses the chimera embryoid body.

A method for cultivating a chimera embryoid body produced by co-cultivating, in the presence of a suitable induction factor, a dental mesenchymal cell and a stem cell of the same mammal species that is selected from all non-human mammal species including a mouse, the stem cell being selected from all types of stem cells including the embryonic stem.

According to a sixth embodiment of the present invention, a method of forming a tooth is provided that involves performing any one of the above methods according and the cultivation method using the chimera embryoid body.

According to an aspect of the above embodiment, a method of forming a tooth in vitro may be provided that involves producing a chimera embryoid body by co-cultivating, in the presence of a suitable induction factor, a dental mesenchymal cell and a stem cell of the same non-human mammal species that is selected from all non-human mammal species including a mouse, the stem cell being selected from all types of stem cells including the embryonic stem cell; and further cultivating the chimera embryoid body to induce formation of secretory cells including those secreting dentin and those secreting enamelin as constituents of a tooth.

According to a seventh embodiment of the present invention, in the above method of forming a tooth in vitro according to the sixth embodiment, the cultivation method involves cultivating the chimera embryoid body for three days on a three dimensional matrix in a serum culture medium that includes none of the induction factors, activin, bone morphogenic protein 4, transforming growth factor β1, insulin growth factor 1, or fibroblast growth factor 2.

According to an aspect of the above embodiment, a method of forming a tooth in vitro may be provided that involves producing a chimera embryoid body by co-cultivating, in the presence of a suitable induction factor, a dental mesenchymal cell and a stem cell of the same non-human mammal species that is selected from all non-human mammal species including a mouse, the stem cell being selected from all types of stem cells including the embryonic stem cell; and further cultivating the chimera embryoid body on a three dimensional matrix without the presence of an induction factor to induce formation of secretory cells including those secreting dentin and those secreting enamelin as constituents of a tooth.

According to an eighth embodiment of the present invention, in the above method of forming a tooth in vitro according to the sixth embodiment, the cultivation method involves cultivating the chimera embryoid body for two days on a three dimensional matrix in a serum culture medium that includes at least one of the inductor factors, activin, bone morphogenic protein 4, transforming growth factor β 1, insulin growth factor 1, or fibroblast growth factor 2; and then cultivating the chimera embryoid body for three days on the three dimensional matrix in a serum culture that includes none of the induction factors.

According to an aspect of the above embodiment, a method of forming a tooth in vitro may be provided that involves producing a chimera embryoid body by co-cultivating, in the presence of a suitable induction factor, a dental mesenchymal cell and a stem cell of the same non-human mammal species that is selected from all non-human mammal species including a mouse, the stem cell being selected from all types of stem cells including the embryonic stem cell; and further cultivating the chimera embryoid body on a three dimensional matrix in the presence of activin, bone morphogenic protein 4, transforming growth factor β 1, insulin growth factor 1, or fibroblast growth factor 2 for two days, and then cultivating the chimera embryoid body on the three dimensional matrix in a serum culture medium that does not any induction factor for three days to induce formation of secretory cells including those secreting dentin and those secreting enamelin as constituents of a tooth.

According to a ninth embodiment of the present invention, a biological matter is provided that is obtained by performing any one of the above methods of forming a tooth in vitro according to the sixth through eighth embodiments.

According to an aspect of the above embodiment, a tooth-like structure including dentin and enamelin may be provided by producing a chimera embryoid body through co-cultivating, in the presence of a suitable induction factor, a dental mesenchymal cell and a stem cell of the same non-human mammal species that is selected from all non-human mammal species including a mouse, the stem cell being selected from all types of stem cells including the embryonic stem cell; and further cultivating the chimera embryoid body on a three dimensional matrix without the presence of an induction factor for three days; or cultivating the chimera embryoid body in a culture medium supplemented with an induction factor for two days, and then cultivating the chimera embryoid body without the induction factor for three days. Further, the present embodiment may be used in regenerative medicine to completely regenerate a tooth by implanting the tooth-like structure into a tooth-extracted portion of a mammal living body, for example.

As is described above, an embodiment of the present invention provides a method of forming a tooth in vitro by producing a chimera embryoid body using an undifferentiated cell and a dental mesenchymal cell of a non-human mammal of the same species, and further cultivating the chimera embryoid body on a three dimensional matrix. Embodiments of the present invention may further be used to provide various techniques related to regenerative medicine utilizing the regeneration mechanism of the living body including techniques for completely regenerating a tooth by implanting in a tooth-extracted portion biological matter according to an embodiment of the present invention that includes dentin and enamelin and is regenerated in vitro; namely, a group of cells secreting the matrices of a tooth-like structure, for example.

### BRIEF DESCRIPTION OF THE DRAWINGS

FIG. 1 is a diagram illustrating a tooth formation process;
FIG. 2 is a diagram illustrating a method of forming a tooth according to an embodiment of the present invention;
FIG. 3 is a diagram illustrating the cultivation of mesenchymal cells separated from the tooth germ of a mouse and the presence/absence of various markers;
FIG. 4 is a diagram illustrating gene expressions and protein expressions of various markers of a cell line used in the present embodiment;
FIG. 5 is a diagram showing phase-contrast microscopic images representing stages of a chimera embryoid body production process;
FIG. 6 is a graph showing gene expression levels of chimera embryoid body samples produced in vitro in the presence of various induction factors;
FIG. 7 is a diagram showing calcium precipitation testing results from examining the chimera embryoid body samples produced in vitro in the presence of various induction factors;
FIG. 8 is a diagram showing calcium precipitation testing results from examining chimera embryoid body samples that were produced in the presence of various induction factors and then transplanted into a living mouse; and
FIG. 9 is a diagram showing protein expressions of various markers in the chimera embryoid body samples that were produced in the presence of various induction factors and then transplanted into a living mouse.

### BEST MODE FOR CARRYING OUT THE INVENTION

In the following, one preferred embodiment of the present invention is described; however, the present invention is by no way limited to such an embodiment.

An embodiment of the present invention relates to a novel method of forming a tooth in vitro in a mammal that involves producing a chimera embryoid body using an undifferentiated cell, which may be any stem cell including an embryonic stem cell (referred to as 'ES cell' hereinafter) that is capable of differentiating into various types of cells and tissues, and a dental mesenchymal cell, and then cultivating the chimera embryoid body on a three-dimensional matrix. It is noted that co-cultivating an ES cell with tissue derived from a living body has been known. However, a method of producing a chimera embryoid body by co-cultivating an ES cell and another cell as in the present embodiment much less techniques involving the use of the chimera embryoid body produced by such a method are not known.

Specifically, a method of forming a tooth in vitro according to an embodiment of the present invention includes a step of co-cultivating an undifferentiated cell and a dental mesenchymal cell derived from a mammal of the same species as that of the target mammal in the presence of an induction factor to produce a chimera embryoid body, and a step of cultivating the chimera embryoid body on a three-dimensional matrix.

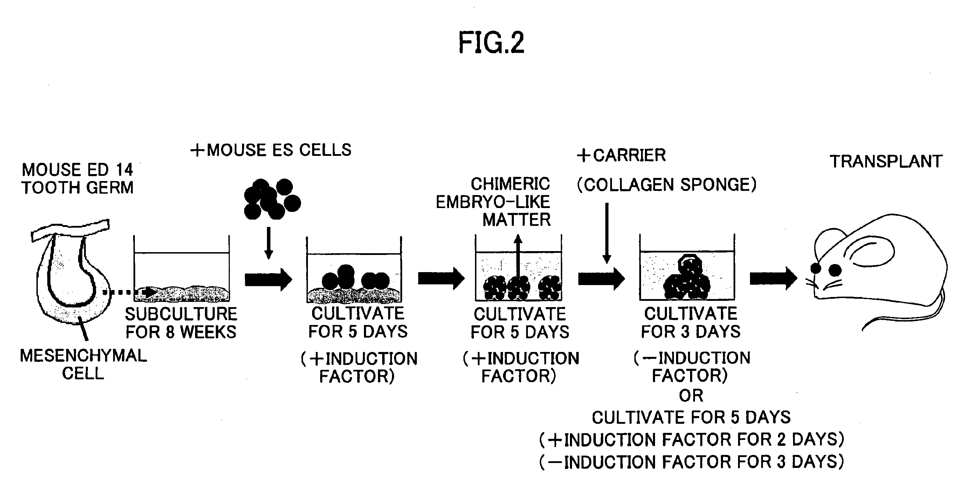

FIG. 2 is a diagram illustrating a method of forming a tooth of a mouse according to an embodiment of the present invention.

As is shown in FIG. 2, according to the present embodiment, first, a dental mesenchymal cell of a 14-embryonal day (ED14) mouse fetus that starts to have differentiation control functions for differentiating into an epithelial cell is extracted and sub-cultured to produce a stable cell line. Then, the cell line is used as feeder cells to be co-cultivated with ES cells of a mouse of the same species in the presence of an induction factor so that a mixed cell group of the dental mesenchymal cells and ES cells of the mouse is produced. Then, the mixed cell group is separated and disseminated in a low binding plastic plate to produce a chimera embryoid body. The process of disseminating the separated cells in a low binding plastic plate involves cultivating the cells in a 96-well plastic plate for one day, and then transferring the cells to a 24-well plastic plate and cultivating the same for four days in the presence of an induction factor. In this way, the step of co-cultivating an undifferentiated cell and a dental mesenchymal cell in the presence of an induction factor to produce a chimera embryoid body may be performed. Then, the step of cultivating the chimera embryoid body on a three dimensional matrix is performed. Specifically, the chimera embryoid body may be disseminated on a three dimensional matrix made of porous collagen and cultivated in a serum culture medium without the induction factor for three days. More preferably the chimera embryoid body may be disseminated on the three dimensional matrix made of porous collagen and cultivated in a culture medium supplemented with an induction factor for two days, and then cultivated in a serum culture medium without an induction factor for three days. As is described above, the present embodiment involves forming a tooth by performing the step of producing an embryo-like matter, and the step of cultivating the embryo-like matter.

In the following, the stem cell used in the present embodiment is described. The stem cell used in the present embodiment is a cell having the capability of differentiating into a specific cell, namely, transforming into a particular cell upon being directed to transform into such a cell, and the capability of retaining its undifferentiated state and reproducing/regenerating itself in such a state over a relatively long period of time. It is noted that the undifferentiated state of a stem cell may be in multiple levels, and at a high level, the stem cell may have high self-reproducing capabilities and be capable of differentiating into a wide range of cells. On the other hand, a stem cell of a lower level may lose its self-reproducing capability and the range of cell lines into which it may differentiate may become limited. The ES cell is a stem cell of a high level that comes after the fertilized egg. The organs of a mammal include tissue specific stem cells (i.e., referred to as somatic stem cells or tissue stem cells). A somatic cell is believed to have the capability of differentiating into a unique cell line and maintaining its tissue by reproducing a cell having the same function as itself upon cell division (self reproduction). It is noted that an embryonic cell (ES cell) may be extracted from an embryo, a somatic cell may be extracted from an adult, and an embryonic germ cell may be extracted from a fetus.

The ES cell is know to have the capability of self-reproducing semi-permanently in an undifferentiated state under certain cultivation conditions and differentiating into any type of cell including germ cells.

The ES cell is extracted from a fertilized embryo, namely, an embryo in its initial stages before a fertilized egg differentiates and develops into a fetus. The ES cell may grow into any cell of the body and is therefore referred to as pluripotent stem cells. The ES cell is extracted from inner cells (inner cell mass) of a blastocyst after 2.5 days from fertilization and is cultivated thereafter. Unlike the somatic stem cell that is extracted from the body, the ES cell is capable of being reproduced indefinitely in the lab through cultivation and has pluripotency, namely, the ability to change into any type of cell. Accordingly, research is being conducted on the ES cell as a material for producing various types of cells as replacement for cells, tissues, or organs that have lost their functions due to accident or disease. Methods for transplanting an organ without causing rejection in the patient may theoretically be devised through applying gene therapy techniques to an ES cell that has been differentiated to alter immune-related genes of the ES cell, or creating an embryo having genetic information of the patient and extracting an ES cell therefrom to induce development of the ES cell into a target cell.

On the other hand, the somatic cell refers to a cell extracted from tissues that have already developed into an organ within the body that is not yet differentiated. An organ includes a large number of cells that have already been differentiated to have specific functions but also includes undifferentiated cells, namely, stem cells that have not yet been differentiated into cells having a particular function. The somatic stem cell has the capability of reproducing a cell identical to itself and differentiating into any particular cell within the organ from which it originates.

Since the somatic cell is known to develop into a specific organ, it is already being used in various therapies, one representative example being the use of bone marrow stem cells in bone marrow transplantation for treating leukemia.

Any of the stem cells having the above-described functions may be used in embodiments of the present invention. In one preferred embodiment, a mesenchymal cell may be extracted from the tooth germ of a lower jaw molar of a 14-embryonal day (ED14) mouse fetus after which the extracted mesenchymal cell is cultivated, and the cultivated mesenchymal cells and ES cells derived from the same type of mouse may be co-cultivated in the presence of an induction factor to produce a chimera embryoid body of the dental mesenchymal cells and ES stem cells of the mouse. However, the stem cell to be used in the present invention is not limited to the ES cell, and any type of stem cell that is capable of inducing generation of the chimera embryoid body as is described above may be used including multipotent stem cells such as the somatic stem cell and the bone marrow stem cell.

Examples of the induction factor that may be used to produce the chimera embryoid body of the dental mesenchymal cells and ES stem cells of a mouse include activin (Ajinomoto), bone morphogenetic protein 4 (BMP-4; Sigma), insulin growth factor 1 (IGF-1; Toyobo), fibroblast growth factor 2 (FGF-2; Toyobo), and transforming growth factor β1 (TGF-β1 ; Toyobo).

Also, in a preferred embodiment, the method for producing the chimera embryoid body though co-cultivation of the dental mesenchymal cells and ES stem cells of a mouse may be performed in the following manner. The ES cells of a mouse are added when the cultivated cells cover approximately 70-80% of a Petri dish, that is, before the dental mesenchymal cells reach confluent growth, and the mixed cells are co-cultivated for approximately five days so that the ES cells may be adequately differentiates. In the process of producing the chimera embryoid body, the cell mixture ratio of the mouse dental mesenchymal cells to the mouse ES cells is arranged to be approximately 2:1. More specifically, in the present embodiment, 8 × 10³ mouse ES cells are provided with respect to 1.7 × 10⁴ cultivated mouse dental mesenchymal cells. After one day of co-cultivation, a MSCGM culture medium supplemented with an induction factor is used to cultivate the mixed cells for a total of five days at 37°C and 5% CO₂. Then, the cells are collected through trypsin and EDTA treatment to be detached into single cells by a plastic filter, and disseminated on a low binding 96-well plastic plate and then a 24-well plastic plate to be cultivated in the presence of the induction factor so that a chimera embryoid body may be produced from the mouse ES cells and the mouse dental mesenchymal cells.

In the following, the method of forming a tooth by further cultivating the chimera embryoid body is described. The chimera embryoid body produced in the above-described manner is cultivated on a three dimensional matrix. For example, the chimera embryoid body may be cultivated on a 1-2 mm × 1-2 mm × 1-2 mm collagen carrier in a serum culture medium without ab induction factor for three days. In a more preferred example, the chimeric embryo-like layer may be cultivated on a 1-2 mm × 1-2 mm × 1-2 mm collagen carrier in a culture medium supplemented with an induction factor for two days, and then cultivated in a serum culture medium without an induction factor for three days.

It has been confirmed through testing that biological matter generated by further cultivating the chimera embryoid body in the above-described manner calcifies on the carrier; expresses genes and proteins of osteopontin, bone sialoprotein, and dental sialoprotein; and further expresses genes and proteins of amelogenin and enamelin of the enamel matrix similar to the tooth formation process illustrated in FIG. 1.

As can be appreciated, in the present embodiment, by producing a chimera embryoid body with mesenchymal cells derived from a mouse fetus tooth germ and ES cells of a mouse and further cultivating the chimera embryoid body on a three dimensional matrix, odontoblast and ameloblast corresponding to secretory cells of dentin and enamelum that make up a tooth may be simultaneously induced. In other words, in the present embodiment, mouse ES cells are differentiated into epithelial cells (ameloblast) by the dental mesenchymal cells thus signifying a first successful case of tooth regeneration.

Also, biological matter obtained by performing the tooth forming method according to an embodiment of the present invention may be used to produce or regenerate a tooth, for example.

In the following descriptions, producing a tooth refers to newly creating a tooth within or outside the living body of a mammal. Also, regenerating a tooth refers to recovering a portion of a tooth that is lost or damaged due to cavity or some other affliction, the recovery taking place within or outside the living body. In other words, the biological matter produced according to an embodiment of the present invention may be used in the so-called regenerative medicine.

It is noted that although an example using a mouse is described herein, the present invention is not limited to such an example and may be applied to all mammals excluding humans.

### (Practical Example)

In the following, a practical example implementing a method according to an embodiment of the present invention is described. However, the present invention is not limited to such an example, and various changes and modifications are possible within the scope of the present invention,

First, establishment of the dental mesenchymal cell line is described.

In the present example, the tooth germ of the lower jaw molars of seven 14-embryonal day (ED14) mouse fetuses were extracted, washed with Hank's solution, and dispersed on ice for four hours to adequately loosen the tissues to thereby separate the mesenchymal cell mass. After performing centrifugal washing for three minutes at 12,000 rpm, the mesenchymal cells were transferred to a plastic dish of 10 cmf and cultivated for one day in α-MEM culture medium (Gibco) supplemented with 10% FBS (fetal bovine serum) at 37 °C and 5% CO₂. The next day, the culture medium was replaced with MSCGM culture medium (Chambrex) and was cultivated in a similar manner at 37 °C and 5% CO₂. Then, the cells were subcultured at the time the cells covered approximately 70-80% of the dish, namely, before the cells reached confluent growth, to create a stable mesenchymal cell line. Commercially available mouse ES-D3 cells (American Type Culture Collection (ATCC)) were disseminated without feeder cells in a 25 mL culture flask coated with 0.1% gelatin beforehand to cultivate the cells while maintaining their undifferentiated states. As the culture medium, KnockOut DMEM culture medium (Gibco) containing 15% KSR (Gibco), 0.1 mMβ-mercaptoethanol (Sigma), 0.1 mM nonessential amino acid (Sigma), 8 mM L-glutamine (Sigma), and 1,000 U/ml Leukemia Inhibitory Factor (LIF; Chemicon) was used and exchanged every other day.

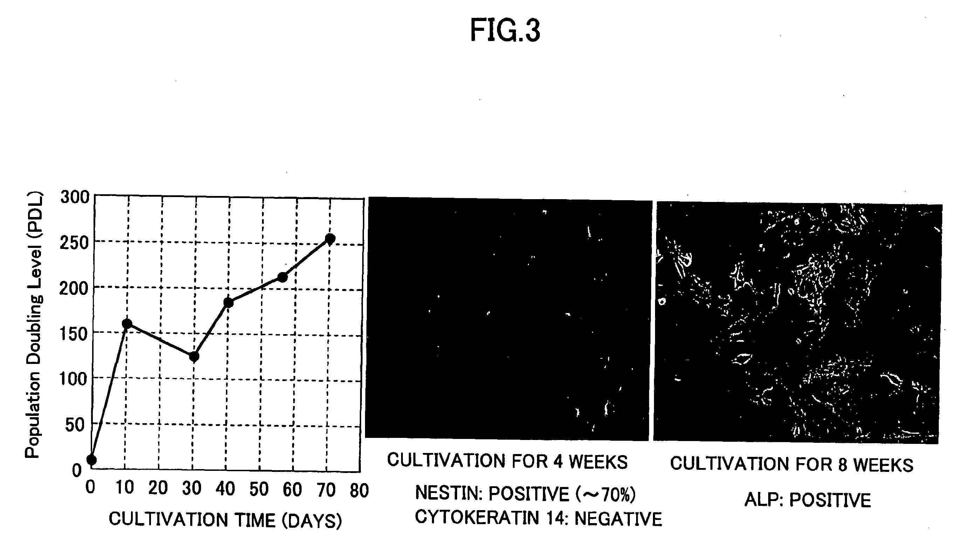

As is shown in the graph on the left side of FIG. 3, the cell line temporarily lost its division capacity after 20 days of cultivation; however, cell division was reactivated after 30 days of cultivation, and a cell line that can be stably subcultured was obtained thereafter. With respect to the cells after four weeks of cultivation (two subcultures), as is shown in the diagram at the center of FIG. 3, it was confirmed that approximately 70% of the cells tested positive for nestin, which corresponds to an undifferentiated cell marker. Also, none of the cells tested positive for cytokeratin 14, which corresponds to a dental epithelial cell marker (not shown). With respect to the cells after eight weeks of cultivation (seven subcultures), the cells still tested positive for nestin (not shown), and as is shown in the diagram on the right side of FIG. 3, it was confirmed that the cells also tested positive for alkali phosphatase (ALP), which corresponds to another undifferentiated cell marker. It may be appreciated from the above test resutls that mesenchymal cells derived from the tooth germ cells that are subcultured for at least 70 days do not contain dental epithelial cells and develop into a cell line with cells maintaining their undifferentiated states, such a cell line being referred to as MDU1 hereinafter.

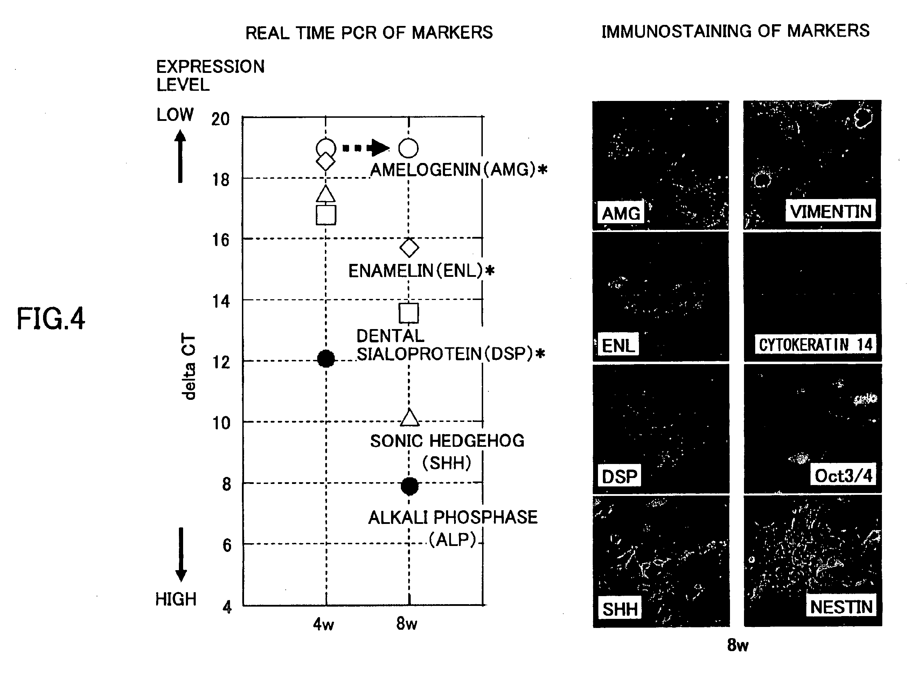

Next, characteristics of the above-described cell line with respect to its gene expression level (Real Time PCR method) and protein expression level (immunostaining) of various markers were examined, the results of which are shown in FIG. 4. As is shown in the graph on the left side of FIG. 4, in the cell line MDU1, the SHH gene expression level of the cells cultivated for eight weeks increased compared to the cells cultivated for four weeks. Also, the ALP gene expression level and the dental sialoprotein (DSP) gene expression level also increased in the cells cultivated for eight weeks compared to those cultivated for four weeks. Also as is shown in the immunostaining images on the right side of FIG. 4, the cells cultivated for eight weeks retained up to 1% of Oct3/4-positive cells and up to 70% of nestin-positive cells corresponding to undifferentiated cell markers, expressed the protein vimentin corresponding to a mesenchymal cell marker, and contained SHH-positive cells, DSP-positive cells, and a small number of AMG-positive and ENL-positive cells. The above testing results show that the cell line MDU1 retains strong mesenchymal cell characteristics even after repeated subcultures, does not lose its undifferentiated characteristic, and shows signs of calcification after long term cultivation, thus signifying that the cell MDU1 is capable of retaining its ability to be differentiated and induced into odontoblast. In turn, it is suggested that this cell line has strong dental induction capabilities.

Also, in addition to the above-described attempt to establish the dental mesenchymal cell line, an attempt was made to subculture the epithelial cells separated from the tooth germ. However, the cell line created from the epithelial cells stopped growing after five days of cultivation to make subculturing impossible. It can be appreciated from such a result that a stable cell line cannot be easily established from the epithelial cells. This may be caused by the fact that when the epithelial cells are separated from the mesenchymal cells, they quickly lose their cell division capabilities and non-induction capabilities. In other words, it may be assumed that mesenchymal cells are necessary for stably cultivating epithelial cells.

Next, the production of a chimera embryoid body from mouse ES cells and the dental mesenchymal cell line is described.

It is understood that a tooth is formed in the living body through interaction between epithelial cells and mesenchymal cells of the tooth germ. Therefore, in considering an in vitro tooth regeneration mechanism, it may be assumed that an epithelial cell line and a mesenchymal cell line derived from the tooth germ that can be stably obtained are necessary. However, as is described above, it is difficult to create a stable cell line with only dental epithelial cells. Also, it is noted that although there have been reports of experiments conducted in an attempt to regenerate a tooth by transplanting a tooth germ itself, a dental epithelium, a dental mesenchymal cell, or a mixed cell group of dental epithelium and dental mesenchymal cells, a completely successful case of tooth regeneration has not yet been reported. Thus, relying on the fact that mesenchymal cells of the tooth germ can be developed into a cell line that can retain undifferentiated cells and epithelial cell support inducing characteristics (e.g., MDU1), the inventor of the present invention tried using mouse ES cells in place of dental epithelial cells.

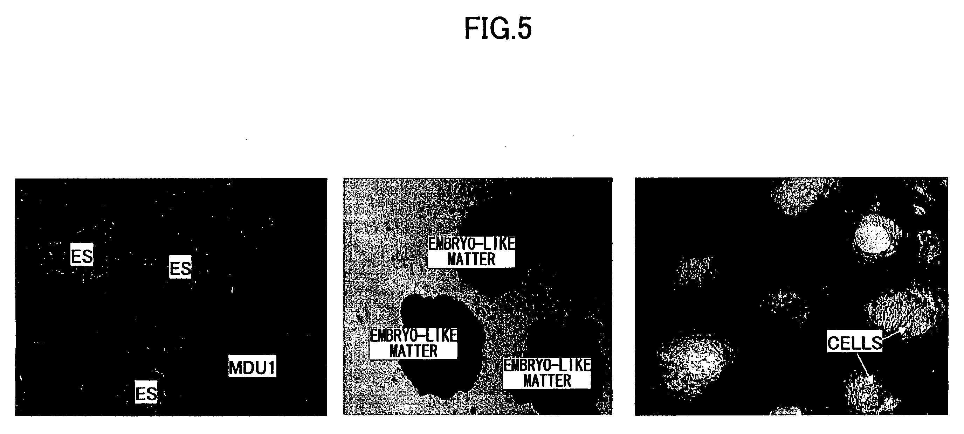

Specifically, as is shown in FIG. 5, mouse ES cells were added to the dental mesenchymal cells at the time the dental mesenchymal cells covered approximately 70-80% of a Petri dish, namely, before the dental mesenchymal cells reached confluent growth, and the dental mesenchymal cells and the ES cells were cultivated for five days so that the ES cells may be adequately differentiated (see left side of FIG. 5). As for the cell mixing ratio, 8 × 10³ mouse ES cells were added with respect to 1.7 × 10⁴ MDU1 cells (see center of FIG. 5). After one day of co-cultivation without an induction factor, a MSCGM culture medium having various types of induction factors added thereto was used to cultivate the mixed cells for a total of five days at 37 °C and 5% CO₂. The induction factors used in the culture medium included activin (Ajinomoto), bone morphogenetic protein 4 (BMP-4; Sigma), insulin growth factor 1 (IGF-1; Toyobo), fibroblast growth factor 2 (FGF-2; Toyobo), and transforming growth factor β1 (TGF-β1; Toyobo). Then, the cells were collected through trypsin-EDTA treatment (Gibco), detached into single cells using a plastic filter, disseminated on a low binding 96-well plastic plate (Nunc) to be cultivated for one day, and then transferred onto a low binding 24-well plastic plate (Nunc) to be cultivated in the presence of the above induction factors for four days to produce the chimera embryoid body. Specifically, by the time the mixed cells of the dental mesenchymal cells and the mouse ES cells have been collected and detached into single cells, and disseminated on the low binding 96-well plastic plate and cultivated for one day, the mixed cells may be round. However, at this stage, the mixed cells are still small and the number of cells is still inadequate so that the mixed cells are transferred to the low binding 24-well plastic plate and supplemented with adequate nutrients (i.e., serum culture medium and induction factors) to cause growth of the cells. The process is ended at the time chimera embryoid body with a diameter of approximately 100-200 µm is produced (i.e., four days of cultivation).

Next, cultivation of the chimera embryoid body on a three dimensional matrix and transplantation of the cultivated biological matter into a living mouse are described.

In one case, the chimera embryoid body produced in the above-described manner was cultivated on a 1-2 mm × 1-2 mm × 1-2 mm collagen carrier (Collagen Sponge Honeycomb (registered trademark)) in a serum culture medium that contains no induction factors for three days. In this case, the chimera embryoid body adhering to the collagen carrier adhered to and extended onto the beam portions of the honeycomb structured collagen carrier (see right side of FIG. 5).

In another case, the chimera embryoid body was cultivated using the same collagen carrier as described above under different cultivation conditions. Specifically, the chimera embryoid body was cultivated in the presence of the above-described induction factors for two days after which they were cultivated in a serum culture medium containing no induction factors for three days. Under such cultivation conditions, the chimera embryoid body by adhering to the collagen carrier adhered to and extended onto the beam portions of the honeycomb structured collagen carrier in a more efficient manner. Then, a portion of the collagen carrier that has absorbed the chimera embryoid body was secured, paraffin-embedded, and stained for observation, and the remaining portions were transplanted under the cranial epithelium and the dorsal fascias of living mouse and then extracted after 42 days to produce a paraffin-embedded sample and an electron-microscope sample. In the following descriptions, the beam portion of the collagen carrier is referred to as scaffold and the void portion of the collagen carrier is referred to as matrix.

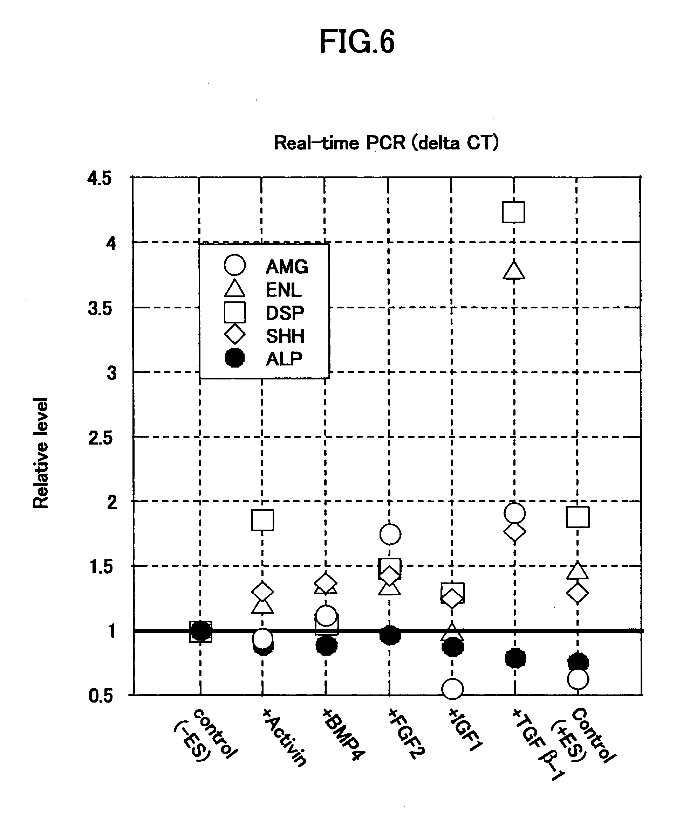

As is shown in FIG. 6, the Real Time PCR method was used to examine the marker gene expressions by the chimera embryoid body. The gene expressions in the chimera embryoid body were compared with those of an embryo-like matter that does not have induction factors added thereto and does not include mouse ES cells (control (-ES)) and an embryo-like matter that does not have induction factors added thereto but includes mouse ES cells (control (+ES)). The level of gene expression for a marker gene within the chimera embryoid body was represented in terms of a relative value with respect to the corresponding gene expression in the control (-ES). As can be appreciated, the gene expression of alkali phosphatase (ALP) fluctuated within a substantially fixed level range so that the number of undifferentiated cells within the dental mesenchymal cells was presumed to be substantially fixed. The gene expressions of amelogenin (AMG), enamelin (ENL), dental sialoprotein (DSP), and sonic hedgehog (SHH) increased by a relatively large extent upon adding TGF-β1. Also, the gene expression for AMG increased upon adding FGF2. Also, the marker gene expressions increased to a certain extent upon adding other inductor factors such as activin, BMP4, and IGF1. The gene expressions also increased by merely adding ES cells to the dental mesenchymal cells. Such testing results suggest that odontoblast formation as well as ameloblast formation may be induced within the chimera embryoid body.

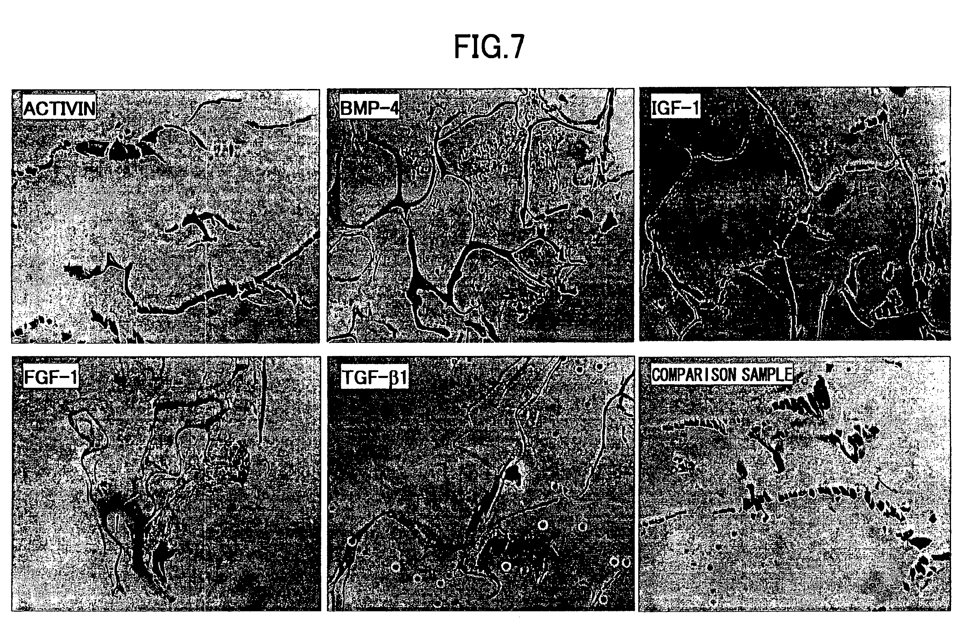

Next, the same samples were subject to examination for odontoblast formation (calcification) using calcium precipitation specific tissue staining (von Kossa-Kernechtrot method), the results of which are illustrated in FIG. 7. In this staining method, cells are stained pink and calcium precipitated portions are stained brown. It is noted that a chimera embryoid body that does not have any inductor factor added thereto was used as a comparison sample. The testing results of the samples including the comparison sample all showed signs of calcium precipitation occurring at the scaffold of the collagen carrier. Particularly, the sample supplemented with IGF-1 showed strong signs of calcium precipitation. It is noted that even in the living body, tooth formation is known to start from calcification of collagen fibers.

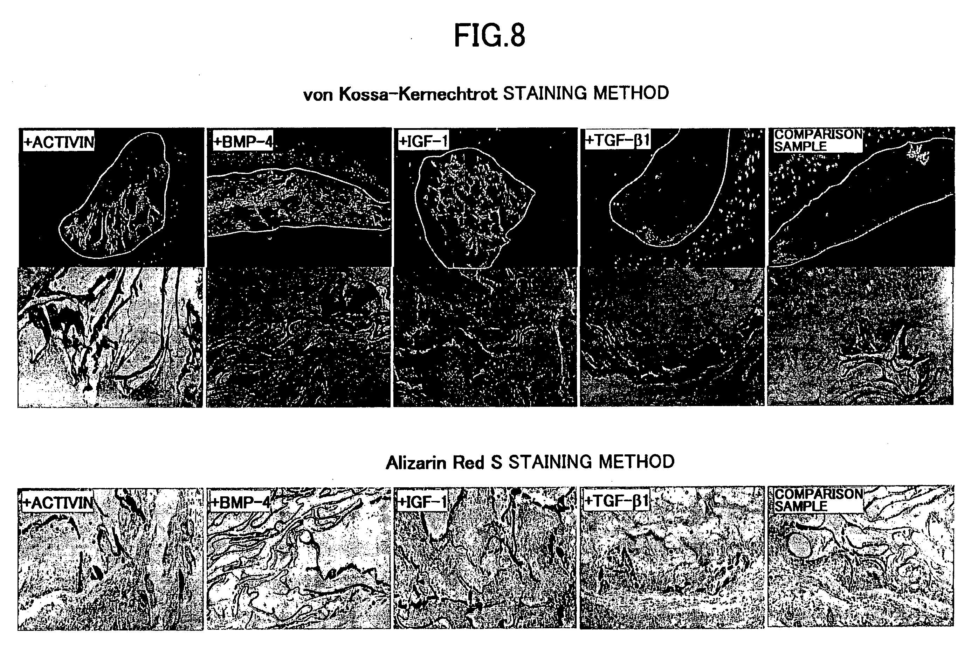

Also, the same samples were transplanted under the cranial epithelium and the dorsal fascias of living mouse and then extracted after 42 days to be subject to examination for the occurrence of calcium precipitation using the von-Kossa-Kernechtrot staining method and the alizarin red S staining method, the results of which are illustrated in FIG. 8. It is noted that in the alizarin S staining method, calcium is stained red, but cells are not stained.

Of the samples examined in the above-described manner, it is noted that the sample having IGF-1 added thereto showed strong signs of calcium precipitation along the scaffold of the collagen carrier as well as within the matrix.

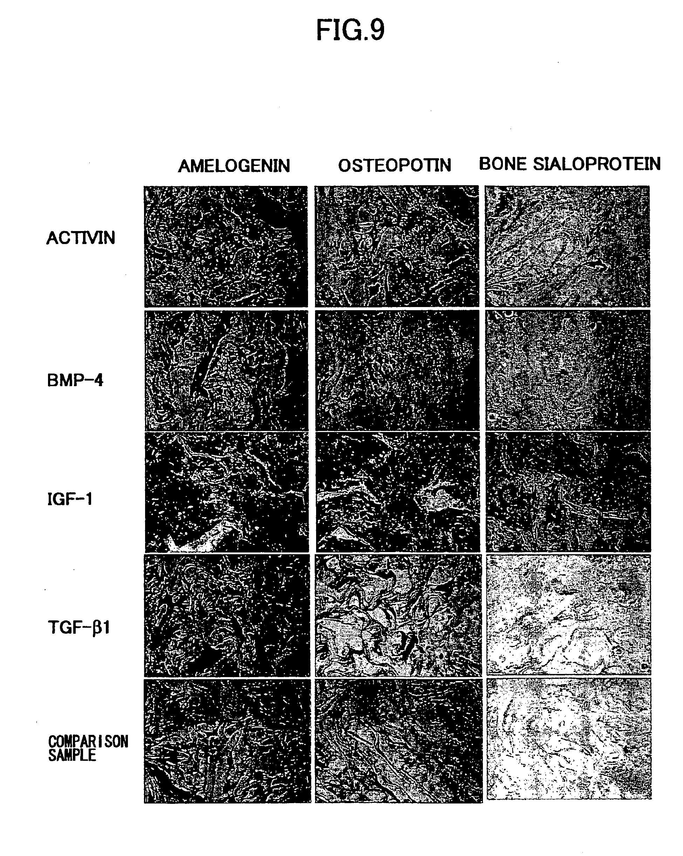

Next, expressions of marker proteins in the samples were examined through immunostaining, the results of which are illustrated in FIG. 9.

As can be appreciated from FIG. 9, the scaffolds of the collagen carriers corresponding to calcified portions were not stained in any of the samples, whereas the matrices of the collagen carriers were stained by amelogenin, ostepotin, and/or bone sialoprotein. Also, cells filled with inorganic substance so that only their nuclei may be seen were observed within the matrices of the collagen carriers of the samples. In the comparison sample, amelogenin and ostepotin were stained along the scaffold corresponding to the calcified portion of the collagen carrier; however, the matrix remained virtually unchanged. The matrices of the collagen carriers that are supplemented with activin, BMP4, or TGF-β1, were stained by amelogenin and ostepotin but showed no signs of staining by bone sialoprotein. Also, the matrix of the collagen carrier supplemented with IGF-1 was prominently stained by amelogenin and ostepotin and slightly stained by bone sialoprotein. It may be appreciated from the above-described testing results that calcification for dentin formation as opposed to that for ossification was occurring in the collagen carrier matrices.

As is described above, in the present example, a chimera embryoid body was produced by creating a cell line through subculture of mesenchymal cells derived from the tooth germ of a mouse fetus, co-cultivating the cell line with mouse ES cells, separating the mixed cells into single cells, and disseminating the separated cells on a lower adhesion plastic plate. Upon further cultivating the chimera embryoid body on a collagen carrier, the chimeric cells adhering to the collagen carrier caused calcification of the scaffold of the collagen carrier, and the cells within the matrix portion of the collagen carrier tested positive for amelogenin thereby implying enamel matrix formation. Also, results of gene expression analysis of the cultivated chimera embryoid body sample indicated increased gene expression levels for amelogenin, enamelin, dental sialoprotein, osteopontin, or bone sialoprotein. Further, actual expressions of the above proteins were confirmed through immunostaining analysis of the sample. In other words, in the present example, secretory cells (i.e., odontoblast and ameloblast) of both dentin and enamel as components of a tooth could be induced from the chimera embryoid body produced using the mesenchymal cells derived from the tooth germ of a mouse fetus and mouse ES cells. Also, it was suggested from this example that the mouse ES cells could be differentiated into epithelial cells (ameloblast) by dental mesenchymal cells. Also, differentiation into a tooth may be further prompted by adding suitable induction factors (e.g., FGF2 or TGF-β1).

It is noted that in the present example, odontoblast and ameloblast as components of a tooth were induced from the chimera embryoid body produced using mesenchymal cells derived from the tooth germ of a mouse fetus and mouse ES cells. However, the present invention is not limited to this example and may be applied to mammals in general. For example, the present invention may equally be applied to tooth regeneration of mammals including humans. Also, undifferentiated cells used in the present invention are not limited to ES cells and stem cells of any type may be used.

Although the present invention is shown and described with respect to certain preferred embodiments, it is obvious that equivalents and modifications will occur to others skilled in the art upon reading and understanding the specification. The present invention includes all such equivalents and modifications, and is limited only by the scope of the claims.

### (Industrial Applicability)

By enabling regeneration of dentin and enamelin using stem cells of mammals, methods may be developed for use in the field of regenerative medicine for regenerating a tooth by filling a hole formed by cavity and the like with corresponding cells and sealing the tooth to induce tooth restoration, or regenerative medicine utilizing the regenerative mechanism of the living body from within to completely regenerate a tooth by implanting cells that secrete matrices of a tooth-like structure into a tooth-extracted portion, namely, implanting biological matter including odontoblast and ameloblast that have been regenerated in vitro into the tooth-extracted portion. Also, the regeneration technique may be applied to a wide range of other fields such as diagnostics for thoroughly diagnosing the tooth of a patient, assay systems, and tests for pharmaceutical development and research, for example. In other words, the present invention has the potential to open new frontiers in the above-described fields and may thus be regarded as having high practical application prospects and industrial utility value.

The present application is based on and claims the benefit of the earlier filing date of Japanese Patent Application No. 2005-221668 filed on July 29, 2005.

## Claims

1. A method of forming a tooth in vitro, comprising: a step of co-cultivating an embryonic stem cell and a dental mesenchymal cell both from a non-human mammal in the presence of an induction factor to produce a chimera embryoid body, the induction factor being at least one selected from activin, bone morphogenic protein 4, insulin growth factor 1, fibroblast growth factor 2, and transforming growth factor β1; and
a step of co-cultivating the chimera embryoid body on a three dimensional matrix in a serum culture medium.

2. A method according to claim 1, wherein, in the step of cultivating the chimera embryoid body, cultivation is for three days on a three dimensional matrix in a serum culture medium that includes none of the induction factors; activin, bone morphogenic protein 4, transforming growth factor β1, insulin growth factor 1, and fibroblast growth factor 2.

3. A method according to claim 1, wherein, in the step of cultivating the chimera embryoid body, cultivation is for two days on a three dimensional matrix in a serum culture medium that includes at least one of the induction factors; activin, bone morphogenic protein 4, transforming growth factor β1, insulin growth factor 1, and fibroblast growth factor 2; and then cultivating the chimera embryoid body for three days on the three dimensional matrix in a serum culture that includes none of the induction factors.

## Patentansprüche

1. Verfahren zum Bilden eines Zahns in vitro, umfassend: einen Schritt des Cokultivierens einer embryonalen Stammzelle und einer dentalen mesenchymalen Zelle, beide von einem nicht-humanen Säuger, in Gegenwart eines Induktionsfaktors, um einen chimären embryoiden Körper zu erzeugen, wobei der Induktionsfaktor mindestens einer, ausgewählt aus Activin, knochenmorphogenetischem Protein 4, Insulinwachstumsfaktor 1, Fibroblastenwachstumsfaktor 2 und transformierendem Wachstumsfaktor β1, ist; und einen Schritt des Cokultivierens des chimären embryoiden Körpers auf einer dreidimensionalen Matrix in einem Serumkulturmedium.

2. Verfahren gemäß Anspruch 1, wobei in dem Schritt des Kultivierens des chimären embryoiden Körpers Kultivierung für drei Tage auf einer dreidimensionalen Matrix in einem Serumkulturmedium geschieht, das keinen von den Induktionsfaktoren Activin, knochenmorphogenetischem Protein 4, transformierendem Wachstumsfaktor β1, Insulinwachstumsfaktor 1 und Fibroblastenwachstumsfaktor 2 einschließt.

3. Verfahren gemäß Anspruch 1, wobei in dem Schritt des Kultivierens des chimären embryoiden Körpers Kultivierung für zwei Tage auf einer dreidimensionalen Matrix in einem Serumkulturmedium geschieht, das mindestens einen von den Induktionsfaktoren Activin, knochenmorphogenetischem Protein 4, transformierendem Wachstumsfaktor β1, Insulinwachstumsfaktor 1 und Fibroblastenwachstumsfaktor 2 einschließt; und dann Kultivieren des chimären embryoiden Körpers für drei Tage auf der dreidimensionalen Matrix in einer Serumkultur, die keinen von den Induktionsfaktoren einschließt.

## Revendications

1. Procédé de formation d'une dent *in vitro,* comprenant:
une étape de co-culture d'une cellule souche embryonnaire et d'une cellule dentaire mésenchymateuse, toutes les deux provenant d'un mammifère non humain, en présence d'un facteur d'induction pour produire un corps embryoïde chimère, le facteur d'induction en étant au moins un sélectionné parmi: activine, protéine 4 morphogénique de l'os, facteur de croissance analogue à l'insuline 1, facteur de croissance des fibroblastes 2 et facteur de croissance transformant β1; et
une étape de co-culture du corps embryoïde chimère sur une matrice tridimensionnelle dans un milieu de culture avec sérum.

2. Procédé selon la revendication 1, dans lequel à l'étape de culture du corps embryoïde chimère, la culture dure trois jours sur une matrice tridimensionnelle dans un milieu de culture avec sérum qui ne comprend aucun des facteurs d'induction; activine, protéine 4 morphogénique de l'os, facteur de croissance transformant β1, facteur de croissance analogue à l'insuline 1 et facteur de croissance des fibroblastes 2.

3. Procédé selon la revendication 1, dans lequel à l'étape de culture du corps embryoïde chimère, la culture dure deux jours sur une matrice tridimensionnelle dans un milieu de culture avec sérum qui comprend au moins l'un des facteurs d'induction: activine, protéine 4 morphogénique de l'os, facteur de croissance transformant β1, facteur de croissance analogue à l'insuline 1 et facteur de croissance des fibroblastes 2; et puis cultiver le corps embryoïde chimère pendant trois jours sur une matrice tridimensionnelle dans une culture avec sérum qui ne comprend aucun des facteurs d'induction.
